# EUROPEAN PATENT SPECIFICATION

(11) **EP 2 415 435 B1**
(45) Date of publication and mention of the grant of the patent: **24.09.2014**
(21) Application number: 10758782.6
(22) Date of filing: 31.03.2010
(51) Int. Cl.: A61F 13/15, A61F 13/472, A61F 13/53, A61F 13/539

(54) **ABSORBENT ARTICLE AND METHOD FOR PRODUCING ABSORBENT ARTICLE**
SAUGFÄHIGER ARTIKEL UND VERFAHREN ZUR HERSTELLUNG DES SAUGFÄHIGEN ARTIKELS
ARTICLE ABSORBANT ET PROCÉDÉ DE PRODUCTION DE L'ARTICLE ABSORBANT

(30) Priority: 31.03.2009 JP 2009085510
(43) Date of publication of application: 08.02.2012
(73) Proprietor: Unicharm Corporation, Ehime 799-0111 (JP)
(72) Inventor: KINOSHITA, Hideyuki, Kanonji-shi Kagawa 769-1602 (JP); KUDO, Jun, Kanonji-shi Kagawa 769-1602 (JP)
(74) Representative: Murgatroyd, Susan Elizabeth
(86) International application number: PCT/JP2010/055823
(87) International publication number: WO 2010/114011

(56) References cited:
- JP-A- 9 108 262
- JP-A- 2006 230 596
- JP-A- 2007 089 819
- JP-A- 2007 152 032
- JP-A- 2007 159 884
- JP-A- 2009 000 173
- JP-B2- 3 406 214
- JP-B2- 4 180 865

## Description

The present invention relates to an absorbent article, and to a method for manufacturing an absorbent article, in which a joint unit that joins at least a topsheet and an absorber together is formed.

### [Background Art]

Conventionally, an absorbent article such as a sanitary napkin or panty liner includes a liquid-permeable topsheet, a liquid-impermeable backsheet, and an absorber provided between the topsheet and backsheet. In such an absorbent article, at least the topsheet and the absorber are joined, specifically, a joint unit is formed by adding pressure (for example, embossing) in the thickness direction of the absorbent article (for example, see Patent Document 1).

The joint unit extends in the front-back direction corresponding to the direction from the wearer's front side (ventral side) toward rear side (dorsal side), that is, in the longitudinal direction of the absorbent article. According to this technology, the joint unit can intercept the wearer's bodily fluids, thus preventing the bodily fluids from leaking out from the absorbent article (so-called side leakage).

However, the conventional absorbent article described above has had problems such as the following. Namely, the joint unit is prone to becoming harder than the periphery of the joint unit, due to being formed by adding pressure in the thickness direction of the absorbent article. In other words, the fact that the joint unit extends along the longitudinal direction of the absorbent article causes the joint unit to become harder, which in turn causes the absorbent article itself to become harder in the longitudinal direction.

For this reason, the absorbent article ends up being difficult to bend from the front side toward the rear side of the wearer, and is thus difficult to fit to the wearer. Therefore, comfort for the wearer when worn is poor and side leakage tends to occur, both of which are problematic.

### [Related Art Document]

### [Patent Document]

[Patent Document1] Japanese Unexamined Patent Application Publication No. H09-108262 (pp. 2-3; Fig. 1 - Fig. 3)

JP-4180865 B2 discloses an absorbent article having a liquid-permeable topsheet, a liquid-impermeable backsheet, an absorbent layer provided between the topsheet and backsheet, and a joint unit formed where the topsheet and absorbent layer are joined. The absorbent layer has a first region having a predetermined weight per unit area of absorbent material and a second region having less weight per unit area of absorbent material, the second region extending along the longitudinal direction of the absorbent article.

According to the present invention, an absorbent article includes a liquid-permeable topsheet, a liquid-impermeable backsheet, and an absorber provided in between the topsheet and the backsheet, such that a joint unit is formed in which at least the topsheet and the absorber are joined, wherein the absorber comprises: a first region having a predetermined weight per unit area of an absorbent material configuring the absorber; and a second region having less weight per unit area of the absorbent material than the first region, the second region extending along the longitudinal direction of the absorbent article; characterized in that the second region is sandwiched between the first regions in the widthwise direction of the absorbent article, and the joint unit is formed in the second region.

### [Brief Description of Drawings]

[Fig. 1] Fig. 1 is a plan view illustrating an absorbent article 1 according to a first embodiment.
[Fig. 2] Fig. 2 is a cross-sectional view (a cross-sectional view taken along the A-A line of Fig. 1) illustrating the absorbent article 1 according to the first embodiment.
[Fig. 3] Fig. 3(a) is an enlarged cross-sectional view illustrating the absorbent article 1 according to the first embodiment, and Fig. 3(b) is a diagram explaining a basis weight of the absorber 40 according to the first embodiment.
[Fig. 4] Fig. 4 is a diagram illustrating the method for manufacturing the absorbent article according to the first embodiment.
[Fig. 5] Fig. 5 is a perspective view illustrating an absorber lamination device 500 according to the first embodiment.
[Fig. 6] Fig. 6(a) to Fig. 6(c) are diagrams illustrating a concave unit 510 according to the first embodiment.
[Fig. 7] Fig. 7 is a plan view illustrating an absorbent article 1A according to a second embodiment.
[Fig. 8] Fig. 8(a) to Fig. 8(c) are cross-sectional views illustrating the absorbent article 1 A according to the second embodiment.
[Fig. 9] Fig. 9 is a plan view illustrating an absorbent article 1B according to a modification (the first thereof).
[Fig. 10] Fig. 10 is a three-side view illustrating only an absorber 40 of an absorbent article 1 B according to the modification (the first thereof).
[Fig. 11] Fig. 11 is a plan view illustrating the absorbent article 1 B according to the modification (the second thereof).
[Fig. 12] Fig. 12 is a three-side view illustrating only the absorber 40 of the absorbent article 1 B according to the modification (the second thereof).

### [Description of Embodiments]

Below are provided descriptions for the absorbent articles and methods for manufacturing the absorbent articles according to the embodiments, with reference to the drawings. In particular, a description is provided for a first embodiment, a second embodiment, a comparative evaluation, and other embodiments.

Also, in the following description of the drawings, identical or analogous parts are predetermined identical or analogous reference numerals. It must be noted that the drawings are schematic ones, and the respective dimensional ratios and the like may differ from reality.

Accordingly, the specific dimensions and the like must be determined by consulting the following description. It is a matter of course that the interrelationships of the drawings also contain parts with mutually differing dimensional ratios and relationships.

### (First Embodiment)

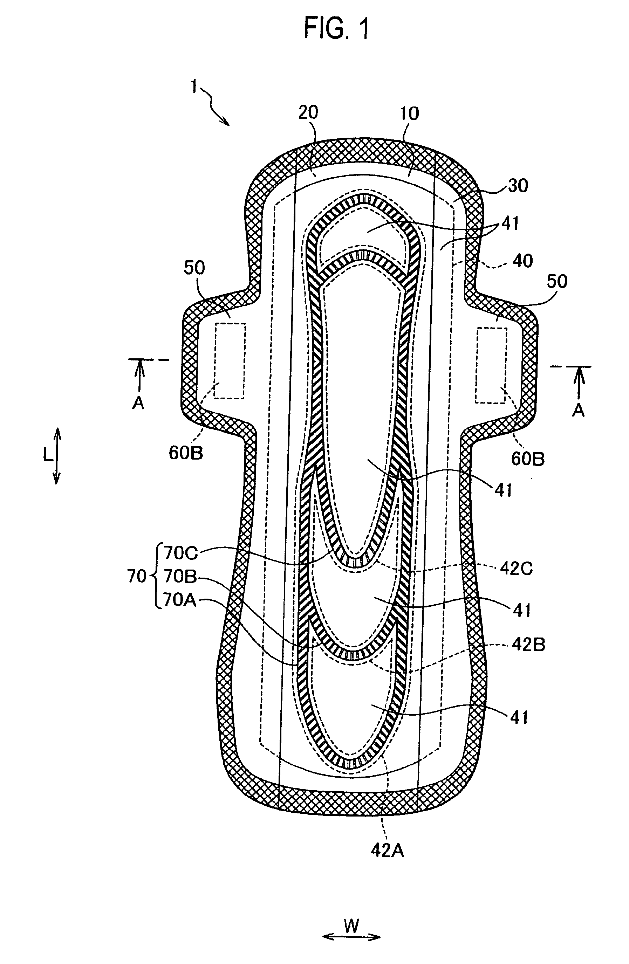

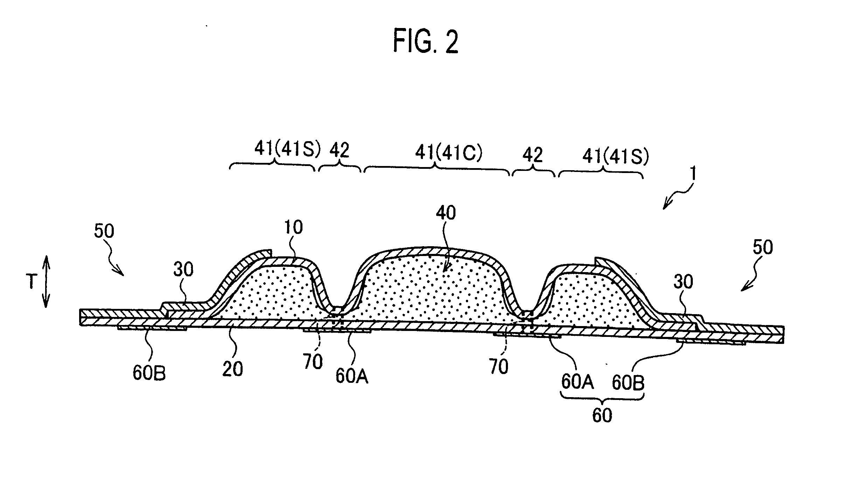

First, a description will be provided with reference to the drawings for the configuration of the absorbent article 1 according to a first embodiment. Fig. 1 is a plan view illustrating the absorbent article 1 according to the first embodiment. Fig. 2 is a cross-sectional view (cross-sectional view taken along the A-A line of Fig. 1) illustrating the absorbent article 1 according to the first embodiment. Note that in the first embodiment, the absorbent article 1 is taken to be a sanitary napkin.

As illustrated in Fig. 1 and Fig. 2, the absorbent article 1 possesses a longitudinally elongated shape in the front-back direction (hereinafter, the "longitudinal direction L" of the absorbent article 1) corresponding to the direction from the wearer's front side (ventral side) toward the rear side (dorsal side).

The absorbent article 1 includes a topsheet 10, a backsheet 20, a side sheet 30, and an absorber 40. A wing unit 50, an adhesive unit 60, and a joint unit 70 are also formed on the absorbent article 1.

The topsheet 10 is provided on the side in contact with the wearer's skin. The topsheet 10 is formed using a liquid-permeable sheet such as air-through non-woven fabric (for example, polyethylene terephthalate and polyethylene).

The backsheet 20 is provided on the non wearer side from the topsheet 10. The backsheet 20 is formed using a liquid-impermeable sheet such as a waterproof film (for example, polyethylene).

The sidesheet 30 is provided on the outer side of the widthwise direction W that is orthogonal to the longitudinal direction L of the absorbent article 1, relative to the topsheet 10. The sidesheet 30 is formed using a liquid-permeable sheet such as a spun bond nonwoven cloth(for example, polypropylene and polyethylene).

The absorber 40 is provided between the topsheet 10 and the backsheet 20. The absorber 40 is formed by covering an absorbent material 40A, made from mixed powder such as a ground pulp or high-water absorbent polymer, with a covering material 40B such as tissue. Note that the absorber 40 will be described in greater detail later.

Herein, in the first embodiment, the above-described topsheet 10, backsheet 20, sidesheet 30, and absorber 40 are respectively bonded by an adhesive agent (for example, a hot melt), thermal fusion bonding or the like.

The wing unit 50 is formed toward the outer side of the widthwise direction W of the absorbent article 1, by extending the backsheet 20 and sidesheet 30 farther than the absorber 40.

The adhesive unit 60 is formed using a member (for example, a hot melt) possessing adhesiveness to be placed in shorts worn by the wearer. The adhesive unit 60 is provided on the opposite-side surface of the topsheet 10 in the backsheet 20.

Specifically, the adhesive unit 60 possesses a long adhesive unit 60A and a short adhesive unit 60B. The long adhesive unit 60A extends along the longitudinal direction L of the absorbent article 1. The short adhesive unit 60B is shorter than the long adhesive unit 60A, and is provided on the portion configuring the wing unit 50 of the backsheet 20.

The joint unit 70 is formed by adding pressure in the thickness direction T of the absorbent article 1 to at least the topsheet 10 and the absorber 40 (for example, by subjecting the same to embossing) so as to join the topsheet 10 and the absorber 40.

The joint unit 70 is in a longitudinally elongated ring shape along the longitudinal direction **L** of the absorbent article 1. Specifically, in the plan view of the absorbent article 1, the joint unit 70 is configured by a large ring unit 70A creating the largest ring shape, a medium ring unit 70B creating a ring shape smaller than that of the large ring unit 70A in the region surrounded by the large ring unit 70A, and a small ring unit 70C creating a ring shape smaller than that of the medium ring unit 70B in the region surrounded by the medium ring unit 70B. The joint unit 70 is formed in a low basis weight region 42 (a first low basis weight region 421), described below, of the absorber 40.

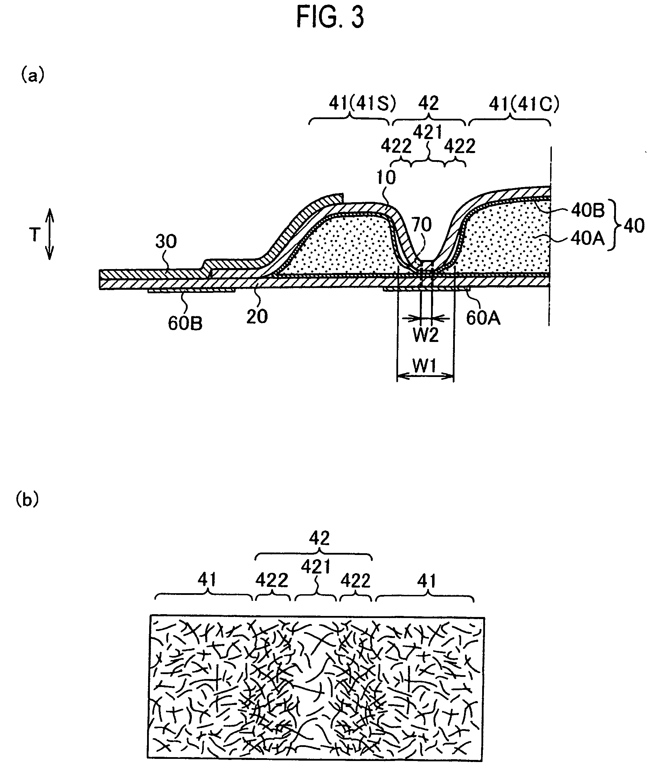

Next a description will be provided with reference to the drawings for the configuration of the above-described absorber 40. Fig. 3(a) is an enlarged cross-sectional view illustrating the absorbent article 1 according to the first embodiment, and Fig. 3(b) is a diagram explaining a basis weight of the absorber 40 according to the first embodiment.

As illustrated in Fig. 3, the absorber 40 is formed using an absorbent material 40A that absorbs the bodily fluids of the wearer, and a covering material 40B that covers the absorbent material 40A. The absorber 40 possesses a high basis weight region 41 (the first region), and the low basis weight region 42 (the second region) provided between the high basis weight regions 41.

The high basis weight region 41 indicates the region other than the low basis weight region 42. The high basis weight region 41 has a predetermined weight per unit area (a so-called basis weight (weight per unit area) (g/m²)) of the absorbent material 40A configuring the absorber 40. The high basis weight region 41 has a higher predetermined basis weight of the absorbent material 40A than the low basis weight region 42.

The high basis weight region 41 is configured by a thick unit 41C positioned in the center of the widthwise direction W and the center of the longitudinal direction L of the absorbent article 1, and a thin unit 41 S formed to be thinner than the thick unit 41C and positioned on the outside of the widthwise direction W of the absorbent article 1 from the thick unit 41C.

The low basis weight region 42 extends along the longitudinal direction L of the absorbent article 1, and extends along the widthwise direction W of the absorbent article 1 (see Fig. 1). The low basis weight region 42 overlaps with the long adhesive unit 60A of the adhesive unit 60 relative to the thickness direction T of the absorbent article 1. In other words, for the above-described joint unit 70, there is also overlap with the long adhesive unit 60A relative to the thickness direction T of the absorbent article 1.

The low basis weight region 42 is in a longitudinally elongated ring shape along the longitudinal direction L of the absorbent article 1. Specifically, in the plan view of the absorbent article 1, the low basis weight region 42 possesses a large ring unit 42A creating the largest ring, a medium ring unit 42B creating a ring smaller than that of the large ring unit 42A in the region surrounded by the large ring unit 42A, and a small ring unit 42C creating a ring smaller than that of the medium ring unit 42B in the region surrounded by the medium ring unit 42B (see Fig. 1).

In other words, because the above-described high basis weight region 41 indicates the region other than the low basis weight region 42, the low basis weight region 42 is provided to be sandwiched between the high basis weight regions 41 in the longitudinal direction L and the widthwise direction W of the absorbent article 1.

The low basis weight region 42 has a lower weight per unit area of the absorbent material 40A than the high basis weight region 41. Specifically, the low basis weight region 42, as illustrated in Fig. 3(b), possesses a first low basis weight region 421 and a second low basis weight region 422.

The above-described joint unit 70 is formed in the first low basis weight region by the application of pressure to at least the topsheet 10 and the absorber 40. On the other hand, no pressure is applied and no joint unit 70 is formed in the second low basis weight region 422.

The joint unit 70 is formed by adding pressure in the thickness direction T of the absorbent article 1 to at least the topsheet 10 and the absorber 40 (for example, by subjecting the same to embossing) so as to join the topsheet 10 and the absorber 40.

Herein, the width (W1) orthogonal to the extending direction of the second low basis weight region 422 is at least equivalent to the width (W2) orthogonal to the extending direction of the joint unit 70. In particular, when the width (W1) is broader than the width (W2), then the relationship X>Z>Y is satisfied, where 'Z' is taken as the density (mass per unit volume (g/cm³)) of the high basis weight region 41, 'X' is taken as the density of the first low basis weight region 421, and 'Y' is taken as the density of the second low basis weight region 422.

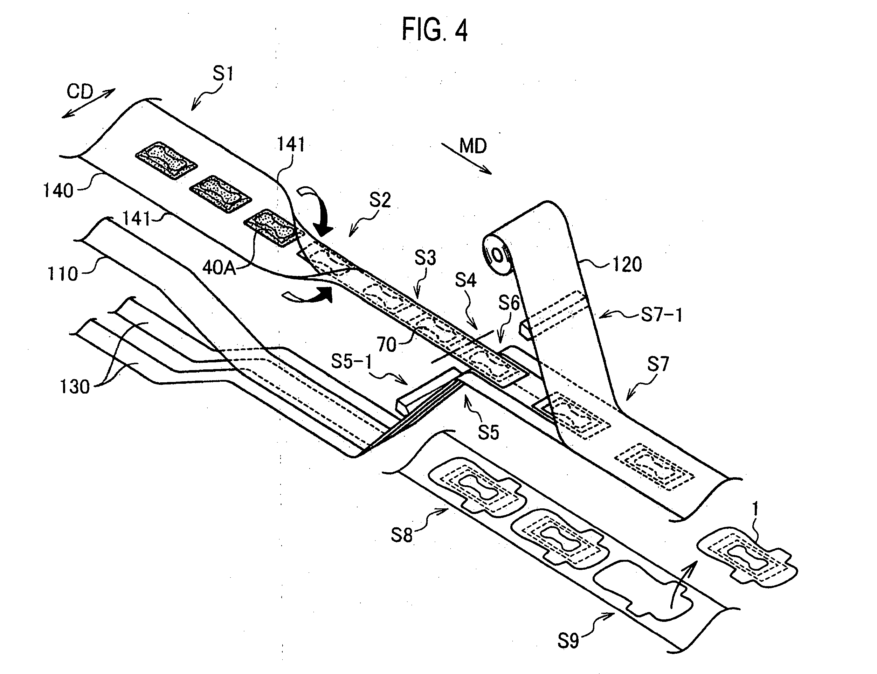

Next, a description will be provided with reference to the drawings for the method for manufacturing the absorbent article according to the first embodiment. Fig. 4 is a diagram explaining the method of manufacturing the absorbent article according to the first embodiment.

As illustrated in Fig. 4, the method for manufacturing the absorbent article includes an absorbent material laminating step S1 (step A), a covering material pleating step S2, a covering material joining step S3, an absorber cutting step S4, an absorber laminating step S5, a joint unit forming step S6 (step B), a backsheet pasting step S7, an article outer shape joining step S8, and an article outer shape cutting step S9.

In the absorbent material laminating step S1, the absorbent material 40A is molded by collecting mixed powder (not shown) such as a ground pulp or a high-water absorbent polymer. At this time, using an absorber lamination device 500 described later, the above-described high basis weight region 41 and the low basis weight region 42 are formed on the absorber 40. Note that the low basis weight region 42 is formed along the longitudinal direction L of the absorbent article 1, and the low basis weight region 42 is formed to be sandwiched by the high basis weight regions 41 in at least the widthwise direction W of the absorbent article 1. Thereafter, the molded absorbent material 40A is laminated at predetermined intervals onto a continuous body 140 of the covering material, such as a tissue that performs continuous conveyance.

In the covering material pleating step S2, both side edges 141 of the crossing direction CD intersecting the conveyance direction MD of the continuous body 140 of the covering material are folded back onto the absorbent material 40A.

In the covering material joining step S3, the continuous body 140 of the covering material covering the absorbent material 40A at predetermined intervals is bounded by adding pressure (for example, by subjecting the same to embossing) in the thickness direction T so as to correspond to the size of the absorber 40 of a single product.

In the absorber cutting step S4, the continuous body 140 of the covering material covering the absorbent material 40A at predetermined intervals is cut to match the size of the absorber 40 of a single product.

In the absorber laminating step S5, the absorber 40 that is cut in the absorber cutting step S4 is laminated onto the continuous body 110 of the topsheet, onto which the continuous body 130 of the sidesheet that is performed continuous conveyance has been pasted. Note that, in S5-1, an adhesive agent (for example, a hot melt) is applied onto the continuous body 110 of the topsheet.

In the joint unit forming step S6, the joint unit 70 is formed by bonding the continuous body 110 of the topsheet with the absorber 40. At this time, the joint unit 70 is formed in the low basis weight region 42.

In the backsheet pasting step S7, the continuous body 120 of the backsheet is pasted onto the continuous body 110 of the topsheet and the continuous body 130 of the sidesheet. The absorber 40 is thereby provided between the continuous body 110 of the topsheet and the continuous body 120 of the backsheet. Note that, in S7-1, an adhesive agent (for example, a hot melt) is applied onto a surface disposed on the absorber 40 side of the continuous body 120 of the backsheet.

In the article outer shape joining step S8, the continuous body 110 of the topsheet and the continuous body 120 of the backsheet, as well as the continuous body 130 of the sidesheet and the continuous body 120 of the backsheet, are respectively bonded together by adding pressure (for example, by subjecting the same to embossing) in the thickness direction T so as to correspond to the outer shape of the absorbent article 1.

In the article outer shape cutting step S9, the continuous body 110 of the topsheet, the continuous body 120 of the backsheet, and the continuous body 130 of the side sheet are cut so as to correspond to the outer shape of the absorbent article 1. The absorbent article 1 is thereby manufactured.

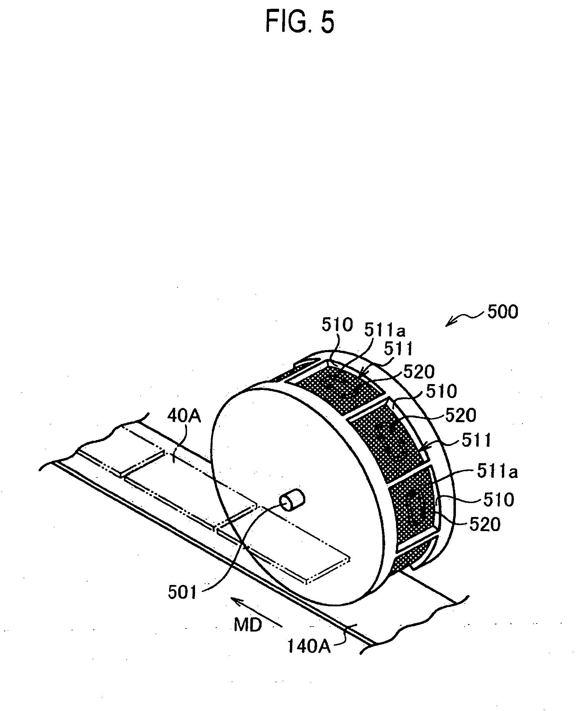

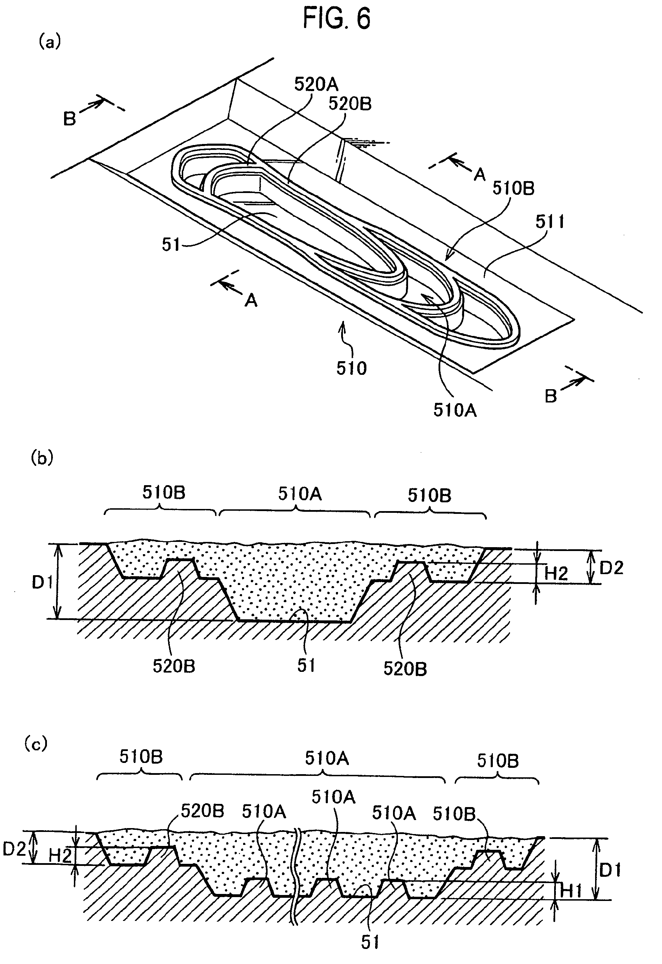

Next, a description will be provided with reference to the figures for the configuration of the absorber lamination device 500 used in the above-described absorbent material laminating step S1. Fig. 5 is a perspective view illustrating the absorber lamination device 500 according to the first embodiment. Fig. 6(a) is a perspective view illustrating a concave unit 510 according to the first embodiment; Fig. 6(b) is a cross-sectional view in the widthwise direction (a cross-sectional view taken along the A-A line of Fig. 6(a)) illustrating the concave unit 510 according to the first embodiment; Fig. 6(c) is a cross-sectional view in the longitudinal direction (a cross-sectional view taken along the B-B line of Fig. 6(a)) illustrating the concave unit 510 according to the first embodiment.

As illustrated in Fig. 5, the absorber lamination device 500 molds the absorbent material 40A from mixed powder sprayed out by means of a spray apparatus (not shown), while rotating around an axial core 501. The absorber lamination device 500 also laminates the molded absorbent material 40A at predetermined intervals onto the continuous body 140 of the covering material that performs continuous conveyance.

The concave unit 510, in which the absorbent material 40A is molded by collecting the mixed powder, is formed on the outer peripheral surface of the absorber lamination device 500. A plurality of mesh-shaped suction holes 511A for sucking the mixed powder is provided on the bottom portion 511 of the concave unit 510. As illustrated in Fig. 6, the concave unit 510 is configured by a deep portion 510A and shallow portion 510B.

The deep portion 510A corresponds to the thick unit 41C. In other words, the deep portion 510A corresponds to the center of the widthwise direction W and the longitudinal direction L of the absorbent article 1. The shallow portion 510B corresponds to the thin unit 41S, and is shallower than the deep portion 510A.

A convex unit 520 corresponding to the low basis weight region 42 is formed on the bottom portion 511 of the concave unit 510. The convex unit 520 possesses a high convex unit 520A and a low convex unit 520B. The high convex unit 520A is formed on the deep portion 510A. The height (H1) of the high convex unit 520A is lower than the depth (D1) of the deep portion 510A. The low convex unit 520B is formed on the shallow portion 510B, and is lower than the high convex unit 520A. The height (H2) of the low convex unit 520B is lower than the depth (D2) of the shallow portion 510B.

In the first embodiment that has been described above, the joint unit 70 is formed in the low basis weight region 42 (the first low basis weight region 421) in which there is less weight per unit area of the absorbent material 40A than that in the high basis weight region 41. Further, the joint unit 70 is formed in the low basis weight region 42 that extends along the longitudinal direction L of the absorbent article 1. Accordingly, the low basis weight region 42 does not become stiffer than the periphery of the joint unit 70 even when pressure is applied to the thickness direction T of the absorbent article 1. In other words, the absorbent article 1 does not become stiff relative to the longitudinal direction L. For this reason, the absorbent article 1 is more easily bent from the wearer's front side toward the rear side (is more flexible), and thus is more easily conformed to the wearer. Side leakage can therefore be more reliably prevented without adversely affecting the comfort when worn for the wearer.

Incidentally, in a case such as when there is a large amount of bodily fluid or when prolonged absorption cannot be avoided, the weight per unit area (basis weight) of the absorbent material is increased in order to ensure the absorption capacity. However, providing the joint unit 70 in an absorbent article 1 in which the absorbent material has a high weight per unit area causes the absorbent article 1 to be prone to becoming stiffer. For this reason, as has been described above, the absorbent article 1 can be maintained flexible by the formation of the joint unit 70 in the low basis weight region 42, and thus is easily fitted to the wearer.

The absorber 40 includes a high basis weight region 41 and low basis weight region 42. Accordingly, compared to when the absorber 40 is formed with only the high basis weight region 41, the total weight of the absorbent material 40A can be reduced since the weight per unit area of the absorber 40A ends up being less. Therefore, it is possible to reduce the costs of manufacturing the absorbent article 1 while also enhancing the performance of the absorbent article 1.

In the first embodiment, the low basis weight region 42 extends both along the longitudinal direction L of the absorbent article 1 and along the widthwise direction W of the absorbent article 1. In other words, the joint unit 70 is also formed along the widthwise direction W of the absorbent article 1. For this reason, it is possible to prevent bodily fluids from the wearer from diffusing to the front or rear side of the wearer. Therefore, it is not only possible to prevent a side leakage but also possible to more reliably prevent bodily fluids from leaking out of the absorbent article 1.

In the first embodiment, the width (W1) orthogonal to the extending direction of the low basis weight region 42 is at least equivalent to the width (W2) orthogonal to the extending direction of the joint unit 70. In particular, the width (W1) is preferably larger than the width (W2). In other words, the relationship X>Z>Y is satisfied, where 'Z' where 'Z' is taken as the density (mass per unit volume (g/cm³)) of the high basis weight region 41, 'X' is taken as the density of the first low basis weight region 421, and 'Y' is taken as the density of the second low basis weight region 422. Accordingly, the bodily fluids absorbed in the high basis weight region 41 are blocked up in the second low basis weight region 422. Therefore, the bodily fluids can be more reliably prevented from leaking out from the absorbent article 1 (in particular, side leakage). In addition, the act of forming the joint unit 70 in the low basis weight region 42 (a so-called productivity of the absorbent article 1) is stable. Note that with the width (W1) being narrower than the width (W2), the joint unit 70 might end up stiffening due to the formation of the joint unit 70 occurring in the high basis weight region 41.

Meanwhile, the low basis weight region 42 is somewhat softer than the high basis weight region 41 because the weight per unit area of the absorbent material 40A in the low basis weight region 42 is lower than that in the high basis weight region 41. Thereby, the absorber 40 gets twisted in the low basis weight region 42. For example, when the low basis weight region 42 is displaced from the long adhesive unit 60A, the movement of the wearer creates a space between the shorts and the absorbent article 1, causing the low basis weight region 42 of the absorber 40 to be prone to twisting.

Therefore, in the first embodiment, the low basis weight region 42 overlaps with the long adhesive unit 60A of the adhesive unit 60 relative to the thickness direction T of the absorbent article 1. In other words, for the above-described joint unit 70, there is also overlap with the long adhesive unit 60A relative to the thickness direction T of the absorbent article 1. Accordingly, the low basis weight region 42 overlapping with the long adhesive unit 60A is secured in the shorts. Therefore, the low basis weight region 42, made to be easily twisted, matches the movement of the wearer (in particular, crotch movements), thus enabling movement with shorts, and enabling the prevention of the absorber 40 from twisting in the low basis weight region 42.

### (Second Embodiment)

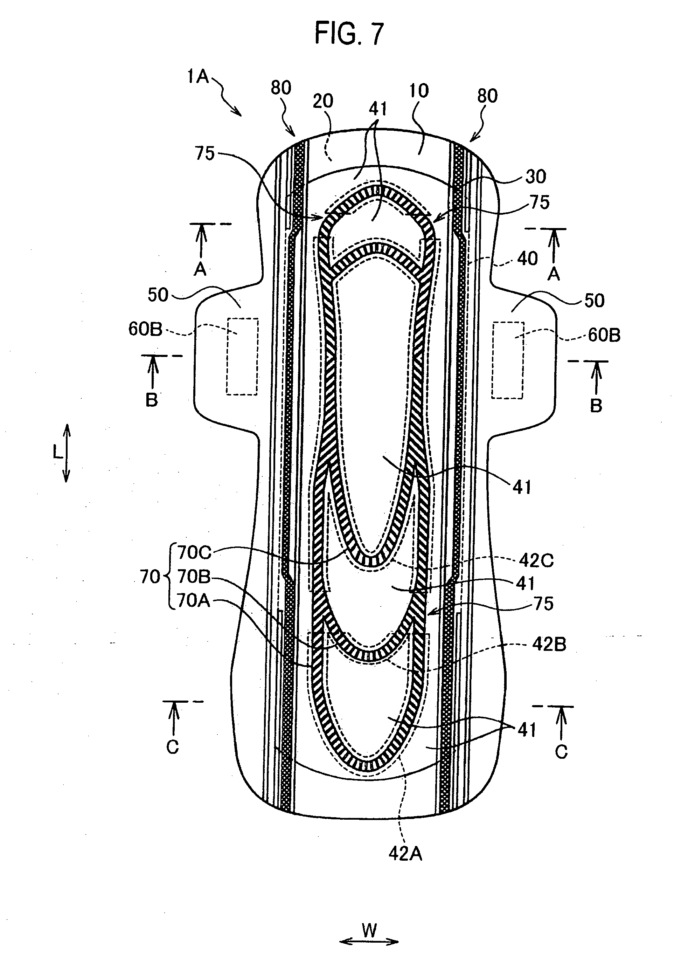

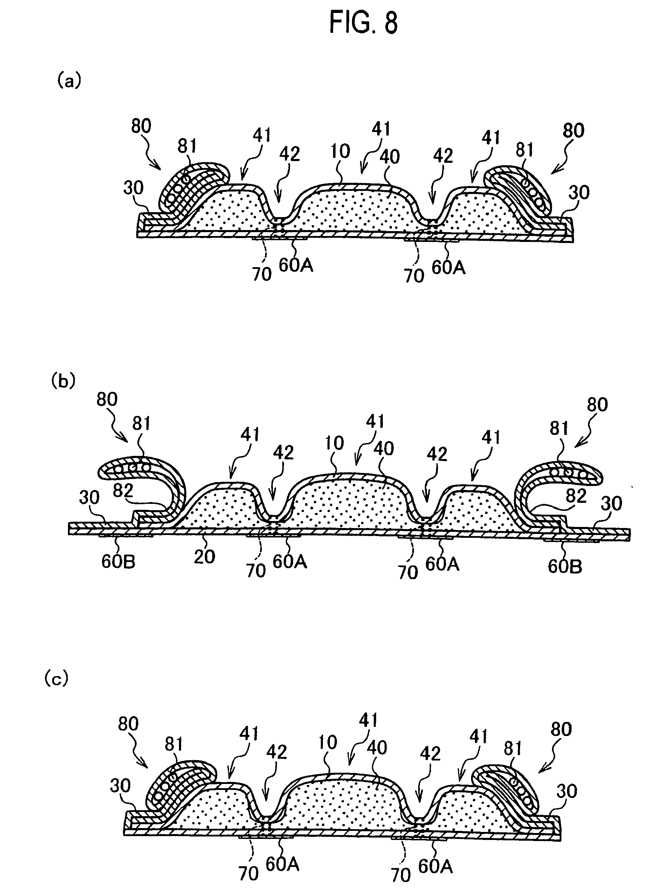

The following is a description provided with reference to the drawings for the configuration of an absorbent article 1A according to a second embodiment. Fig. 7 is a plan view illustrating the absorbent article 1A according to the second embodiment. Fig. 8(a) is a cross-sectional view (cross-sectional view taken along the A-A line of Fig. 7) illustrating the absorbent article 1A according to the second embodiment; Fig. 8(b) is a cross-sectional view (cross-sectional view taken along the B-B line of Fig. 7) illustrating the absorbent article 1A according to the second embodiment; and Fig. 8(c) is a cross-sectional view (cross-sectional view taken along the C-C line of Fig. 7) illustrating the absorbent article 1A according to the second embodiment. Note that the description focuses primarily on the points of difference, and the same reference numerals are assigned to the same portions as in the absorbent article 1 according to the above-described first embodiment.

In the first embodiment described above, the absorbent article 1 includes the topsheet 10, the backsheet 20, the sidesheet 30, and the absorber 40. By contrast, in the second embodiment, instead of the sidesheet 30, the absorbent article 1A includes a leakage-preventing unit 80 formed by extending the sidesheet 30 in the widthwise direction W of the absorbent article 1A.

Specifically, as illustrated in Fig. 7 and Fig. 8, the leakage-preventing unit 80 is provided along the longitudinal direction L of the absorbent article 1A, at the end sides of the absorber 40. A cord-like body 81 made of rubber or the like is provided in the leakage-preventing unit 80 and possesses elasticity in the longitudinal direction L of the absorbent article 1A. The cord-like body 81 is disposed along the longitudinal direction L of the absorbent article 1A, while extending in the longitudinal direction L of the absorbent article 1A in the folded-back leakage-preventing unit 80. The leakage-preventing unit 80 thereby rises (so-called solid gather) in the state in which the absorbent article 1A has been worn on the wearer.

The low basis weight region 42 is not provided in the portion 75 to which the large ring unit 70A and medium ring unit 70B of the joint unit 70 are adjacent. Rather, the low basis weight region 42 is preferably provided at least in the vicinity of the point of discharge (that is, the small ring unit 42C) where the bodily fluids of the wearer are discharged.

The low basis weight region 42 (that is, the first low basis weight region 421 and the second low basis weight region 422) does not overlap with the leakage-preventing unit 80 relative to the thickness direction T of the absorbent article 1A. In particular, the low basis weight region 42 preferably does not overlap with a standing base point 82 where the leakage-preventing unit 80 stands up relative to the thickness direction T. Therefore, regarding the joint unit 70 as well, there is no overlap with the leakage-preventing unit 80 relative to the thickness direction T of the absorbent article 1.

In the second embodiment described above, the low basis weight region 42 and the joint unit 70 do not overlap with the inside of the leakage-preventing unit 80 relative to the thickness direction T of the absorbent article 1A. Accordingly, as there is no overlap, the base point receives stress occurring due to the standing up of the leakage-preventing unit 80 (that is, the standing base point 82) with the low basis weight region 42. For this reason, the standing of the leakage-preventing unit 80 can be prevented from becoming unstable. Side leakage can therefore be more reliably prevented without adversely affecting the comfort for the wearer when worn. Note that when the low basis weight region 42 and the joint unit 70 do end up overlapping with the standing base point 82, the low basis weight region 42 may be prone to twisting due to the stress from the standing of the leakage-preventing unit 80.

In particular, the low basis weight region 42 is preferably provided at least in the vicinity of the point of discharge where the bodily fluids of the wearer are discharged. Accordingly, the absorbent article 1A is easily fitted to the wearer, because the vicinity of the point of discharge (a so-called crotch area) becomes the most bent region when the absorbent article 1A is worn by the wearer. Side leakage can therefore be more reliably prevented without adversely affecting the comfort for the wearer when worn.

### (Modification)

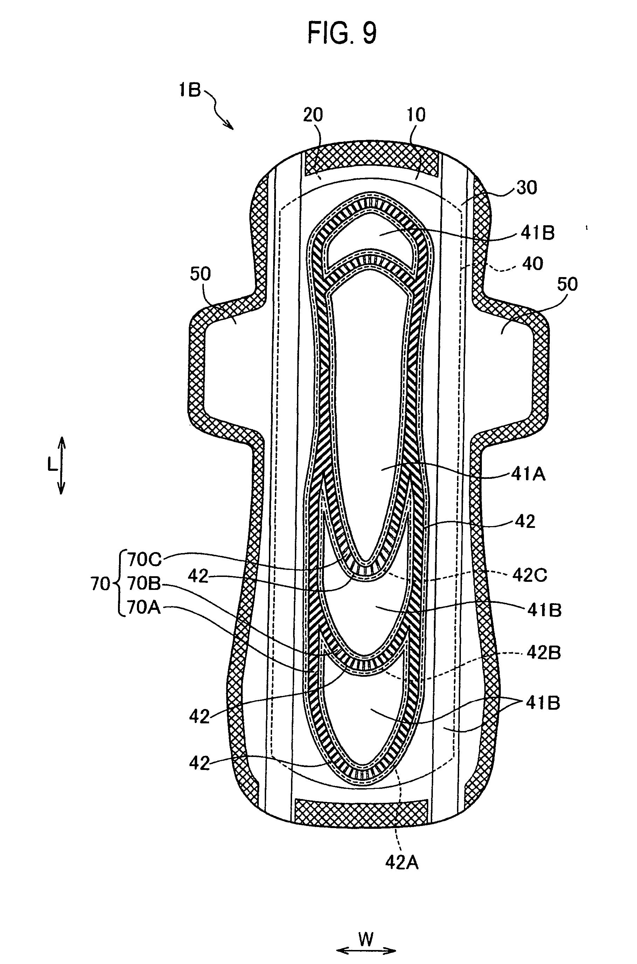

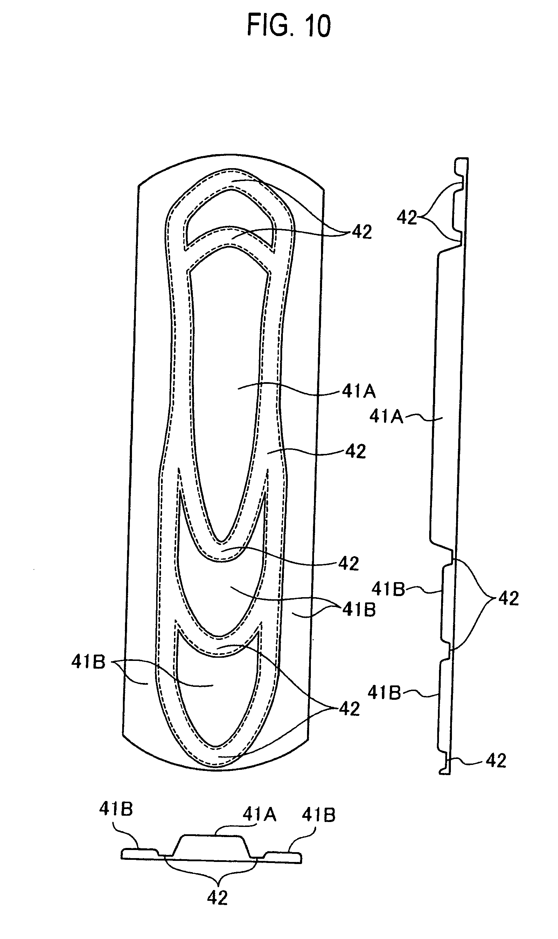

The absorbent article 1A according to the second embodiment described above may be modified as follows. Fig. 9 is a plan view illustrating an absorbent article 1 B of a modification. Fig. 10 is a three-side view illustrating only the absorber 40 of the absorbent article 1 B according to the modification. Note that a description is provided by focusing on the points of difference, and the same reference numerals are assigned to the same portions as in the absorbent article 1 A according to the second embodiment described above.

In the second embodiment described above, like in the first embodiment, the high basis weight region 41 has entirely the same configuration in those regions other than the low basis weight region 42. By contrast, in the modification, the high basis weight region 41 is configured by an inner high basis weight region 41A (the inner region) and an outer high basis weight region 41 B (the outer region).

As illustrated in Fig. 9 and Fig. 10, the inner high basis weight region 41 A is positioned farther inward than the low basis weight region 42, relative to the longitudinal direction L and widthwise direction W of the absorbent article 1. Specifically, the inner high basis weight region 41A faces the point of discharge where the bodily fluids of the wearer are discharged, and is provided at a position in contact with the point of discharge (in other words, in the small ring unit 42C). The basis weight of the inner high basis weight region 41 A is, for example, 500 to 1000 g/m².

Note that the inner high basis weight region 41A need not necessarily be positioned farther inward than the low basis weight region 42 relative to the longitudinal direction L and widthwise direction W of the absorbent article 1, but may be positioned farther inward than the low basis weight region 42 relative to at least the widthwise direction W of the absorbent article 1.

The outer high basis weight region 41 B is positioned farther outward than the low basis weight region 42 relative to the longitudinal direction L and widthwise direction W of the absorbent article 1, and has a lower weight per unit area of the absorbent material 40A than the inner high basis weight region 41A. Specifically, the outer high basis weight region 41 B is provided at the position where the small ring unit 42C and the low basis weight region 42 are excluded. The basis weight of the outer high basis weight region 41 B is, for example, 300 to 600 g/m².

Note that the outer high basis weight region 41 B need not necessarily be positioned farther outward than the low basis weight region 42 relative to the longitudinal direction L and widthwise direction W of the absorbent article 1, and may be positioned farther outward than the low basis weight region 42 relative to at least the widthwise direction W of the absorbent article 1.

Herein, the low basis weight region 42 extends along the longitudinal direction L of the absorbent article 1. Further, the low basis weight region 42 extends along the widthwise direction W of the absorbent article 1, that is, along the medium ring unit 42B and the small ring unit 42C. The basis weight of the low basis weight region 42 is, for example, 50 to 200 g/m².

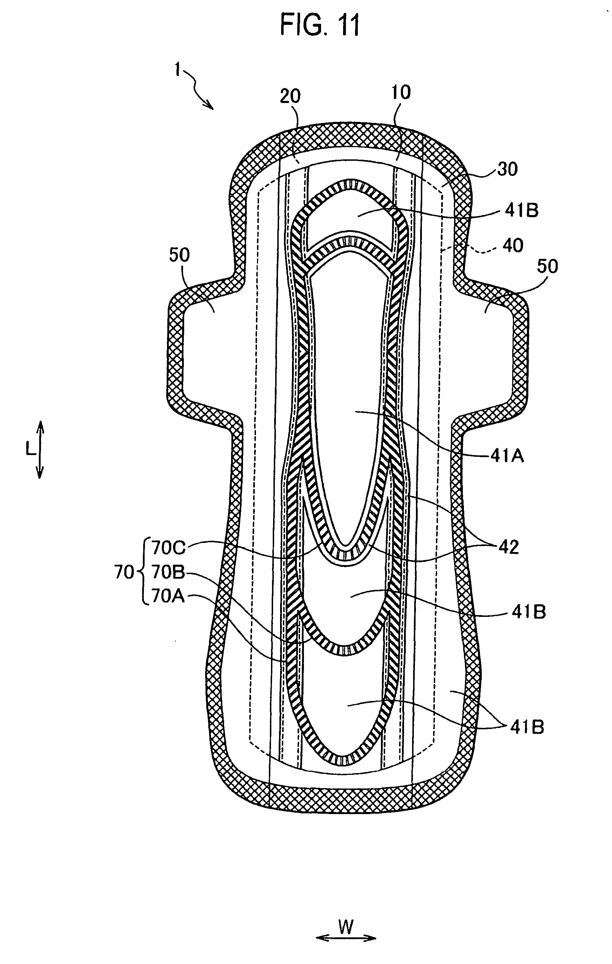

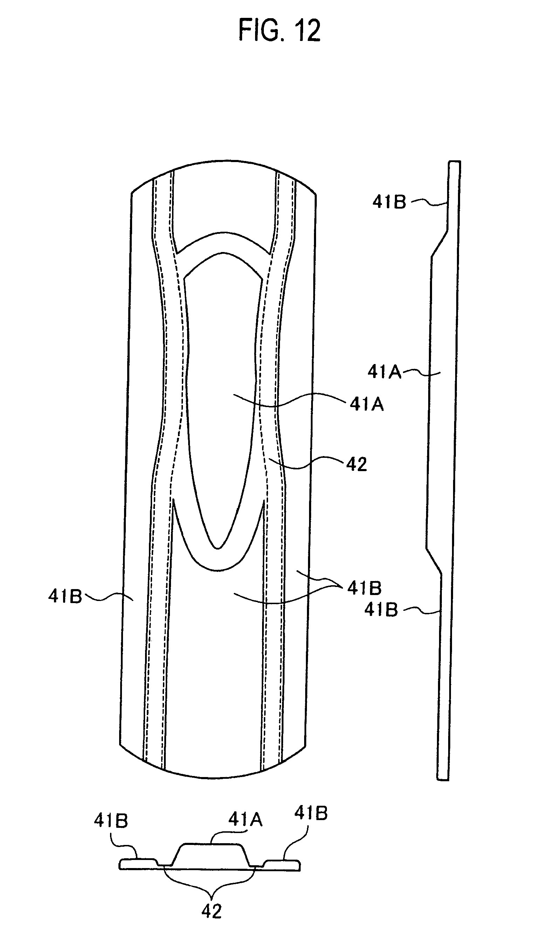

The joint unit 70 may be provided in at least some of the low basis weight region 42. For example, as illustrated in Fig. 11 and Fig. 12, the low basis weight region 42 may extend along only the outside of the small ring unit 42C relative to the widthwise direction W of the absorbent article 1, rather than along the large ring unit 42A or the medium ring unit 42B relative to the widthwise direction W of the absorbent article 1.

### [Comparative Evaluation]

The following is a description of the results of the tests conducted using the absorbent articles according to the following comparative example and example, provided in order to further clarify the effects of the embodiments. Specifically, the evaluations for bending stiffness and diffusiveness are described. Note that the embodiments are in no way restricted by these examples.

First, the configurations of the absorbent articles according to the comparative example and the example are as shown in Table 1. Note that in each evaluation, a test piece was used that was formed by cutting the absorbent articles according to the comparative example and example such that two joint units along the longitudinal direction L of the absorbent article were included. Also, the conditions of the test pieces are as shown in Table 1.

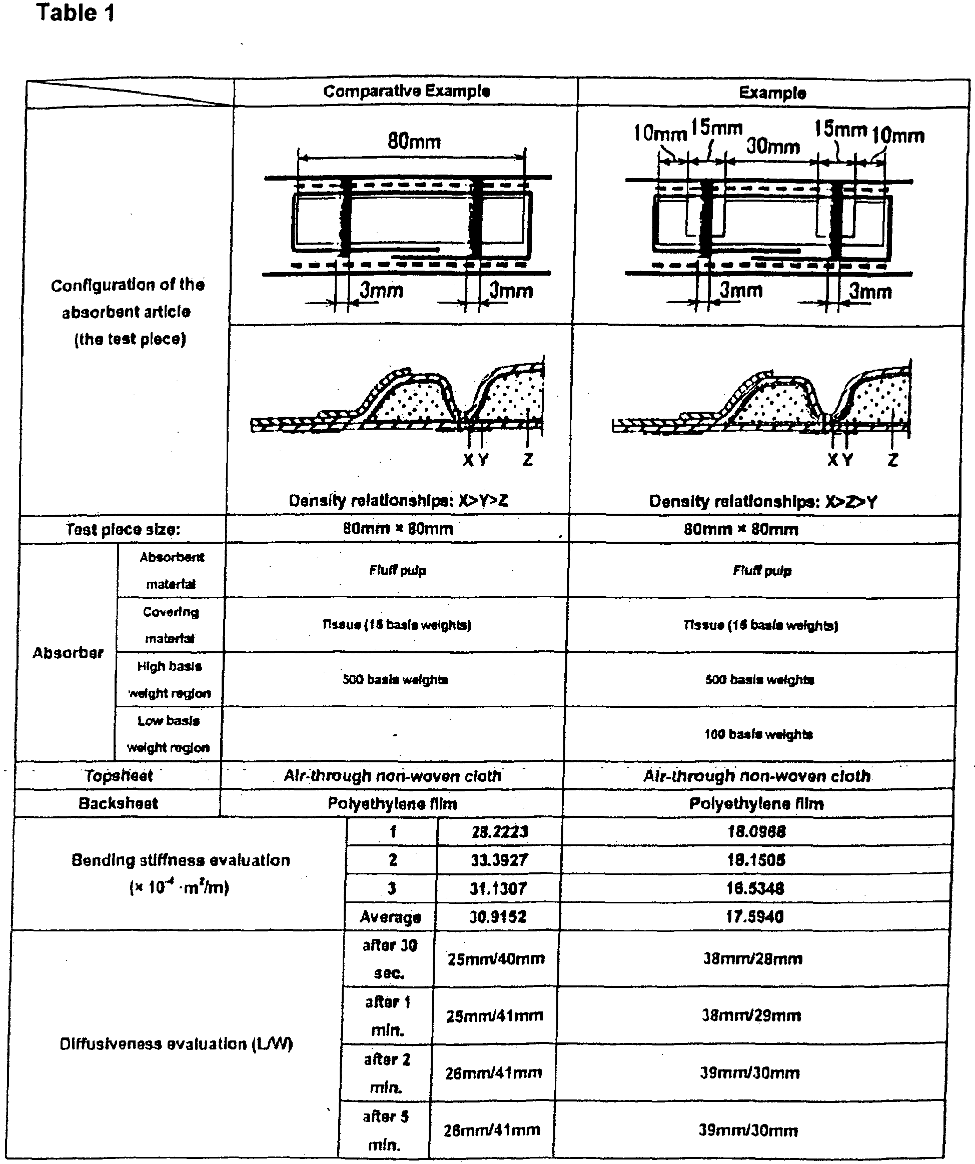

### (Evaluation of Bending Stiffness)

The bending stiffness evaluation indexed values for the bending stiffness relative to the longitudinal direction L of each test piece, for example, using the KES-FB2-L large pure bending tester manufactured by KATO TECH CO., LTD. The value for the bending stiffness is the stiffness value at the midway curvature K = -0.1 to -0.3 cm⁻¹ and K = 0.1 to 0.3 cm⁻¹ when a curvature K=-0.5 to 0.5 cm⁻¹ was taken as one cycle and each absorbent article was folded in each segment. Note that the smaller the index, the softer relative to the longitudinal direction L of the absorbent article.

The results, as shown in Table 1, found that the absorbent article according to the example was softer relative to the longitudinal direction L of the absorbent article when compared to the absorbent article according to the comparative example.

Also, the evaluated index was found to preferably be 35 to 5 (x 10-4 N.m²/m), and in particular even more preferably be 20 to 10 (× 10-4 N·m²/m). Note that when the index is greater than 35, it becomes difficult to fit to the wearer due to stiffening relative to the longitudinal direction L of the absorbent article. On the other hand, when the index is less than 5, in cases when the leakage-preventing unit was provided, the absorbent article was unable to withstand the stress of the cord-like body, and the standing of the leakage-preventing unit became unstable, leading to the occurrence of twisting in the absorbent article.

### (Evaluation of Diffusiveness)

The diffusiveness evaluation indexed the diffusion state after 30 seconds, 1 minute, 2 minutes, and 5 minutes, when 5cc of artificial menstrual blood were dripped onto the point in contact with the point of discharge of each absorbent article (in other words, inside the small ring unit 42C) to spend for 20 seconds. The length of diffusion in the longitudinal direction L of the absorbent article from the point where the artificial menstrual blood was dripped (the menstrual blood dripping point) and the length of diffusion in the widthwise direction W of the absorbent article from the menstrual blood dripping point were indexed as the diffusion state. Note that the higher the number, the more excellent the diffusiveness.

The results, as shown in Table 1, show that the absorbent article according to the example was more easily able to diffuse toward the longitudinal direction of the absorbent article while also preventing diffusion in the widthwise direction of the absorbent article, when compared to the absorbent article according to the comparative example. In other words, the absorbent article according to the example was found to be able to more reliably prevent side leakage by more easily diffusing the menstrual blood in the longitudinal direction of the absorbent article in the high basis weight region 41, when compared to the absorbent article according to the comparative example.

### (Other Embodiments)

As has been described above, the content of the present invention has been disclosed by means of the embodiments, but the statements and drawings, which form a part of this disclosure, must not be understood as limiting the present invention. The various alternative modes of carrying out the present invention, embodiments and operational techniques will be apparent to the person having ordinary skill in the art based on this disclosure.

For example, the embodiments can be modified as follows. Specifically, although the absorbent article 1 has been described as being a sanitary napkin, there is no limitation thereto, and a panty liner, a disposable diaper and the like may be used. In other words, there is no particular limitation to the configuration of the absorbent article 1 (for example, the absorber 40, the joint unit 70, and the leakage-preventing unit 80), which can be appropriately selected depending on the purpose.

Further, it is a matter of course that the method for manufacturing the absorbent article 1 is not to be limited to what was described in the first embodiment described above, and may be appropriately selected according to the purpose, provided that at least the absorbent material laminating step S1 and the joint unit forming step S6 are performed.

Moreover, although the low basis weight region 42 has been described as being provided to be sandwiched by the high basis weight regions 41 in the longitudinal direction L and widthwise direction W of the absorbent article 1, there is no limitation thereto, and the same may be provided to be sandwiched by the high basis weight regions 41 in at least the widthwise direction W of the absorbent article 1.

Further, the low basis weight region 42, although having been described as overlapping with the long adhesive unit 60A of the adhesive unit 60 relative to the thickness direction T of the absorbent article 1, is not limited thereto, and may be made not to overlap with the long adhesive unit 60A.

In addition, in the absorber lamination device 500, although the provided description was that the high basis weight region 41 and the low basis weight region 42 are formed by the convex unit 520 provided in the bottom portion 511 of the concave unit 510, there is no limitation thereto, and the high basis weight region 41 and the low basis weight region 42 may be formed by modifying the size or intervals of the suck holes 511 A.

In this manner, the present invention, of course, includes various modes and the like that have not be recited herein. Therefore, the technical scope of the present invention is to be defined only by the specific inventive items according to the claims validated from the above description.

### [Industrial Applicability]

According to the features of the present invention, when the joint unit is provided, it is possible to provide an absorbent article and a method for manufacturing the absorbent article capable of more reliably preventing side leakage without adversely affecting the comfort for the wearer when worn.

## Claims

1. An absorbent article comprising a liquid-permeable topsheet (10), a liquid-impermeable backsheet (20), and an absorber (40) provided in between the topsheet (10) and the backsheeet (20), such that a joint unit (70) is formed in which at least the topsheet (10) and the absorber (40) are joined, wherein the absorber (40) comprises:
a first region (41) having a predetermined weight per unit area of an absorbent material configuring the absorber (40); and
a second region (42) having less weight per unit area of the absorbent material than the first region (41),
the second region (42) extending along the longitudinal direction of the absorbent article,
**characterized in that** the second region (42) is sandwiched between first regions (41) in the widthwise direction of the absorbent article, and the joint unit (70) is formed in the second region (42).

2. The absorbent article according to claim 1, wherein the second region (42) extends along the widthwise direction of the absorbent article.

3. The absorbent article according to claim 1, wherein a width (W1) orthogonal to the extending direction of the second region (42) is at least equivalent to a width (W2) orthogonal to the extending direction of the joint unit (70).

4. The absorbent article according to claim 1, further comprising a leakage-preventing unit (80) disposed along the longitudinal direction of the absorbent article with a cord-like body (81) possessing elasticity in the longitudinal direction of the absorbent article stretched at the end sides of the absorber (40), wherein the second region (42) does not overlap with the leakage-preventing unit (80) relative to the thickness direction of the absorbent article.

5. The absorbent article according to claim 1, wherein an adhesive unit (60) possessing adhesiveness is provided on the opposite-side surface of the topsheet (10) in the backsheet (20), and the second region (42) overlaps with the adhesive unit (60) relative to the thickness direction of the absorbent article.

6. The absorbent article according to claim 1, wherein the first region (41) comprises:
an inner region (41A) positioned farther inward than the second region (42) relative to the widthwise direction of the absorbent article; and
an inner region (41 B) positioned farther outward than the second region (42) relative to the widthwise direction of the absorbent article, and having less weight per unit area of the absorbent material than the inner region (41A)

7. A method for manufacturing an absorbent article that comprises a liquid-impermeable topsheet (10), a liquid-impermeable backsheet (20), and an absorber (40) provided in between the topsheet (10) and the backsheet (20), the method comprising:
a step A of forming in the absorber (40) a first region (41) having a predetermined weight per unit area of an absorbent material configuring the absorber (40) and a second region (42) having less weight per unit area of the absorbent material than the first region (41); and
a step B of forming a joint unit (70) by joining at least the topsheet (10) and the absorber(40), wherein
in the step A, the second region (42) is formed along the longitudinal direction of the absorbent article, and
the method being **characterized in that**:
the second region (42) is formed to be sandwiched between first regions (41) in the widthwise direction of the absorbent article, and
in the step B, the joint unit (70) is formed in the second region (42).

## Patentansprüche

1. Saugfähiger Artikel, umfassend eine flüssigkeitsdurchlässige Deckschicht (10), eine flüssigkeitsundurchlässige Rückschicht (20) und einen Saugkörper (40), der zwischen der Deckschicht (10) und der Rückschicht (20) vorgesehen ist, so dass eine Verbundeinheit (70) ausgebildet wird, bei der mindestens die Deckschicht (10) und der Saugkörper (40) miteinander verbunden sind, wobei der Saugkörper (40) umfasst:
einen ersten Bereich (41), der ein vorbestimmtes Gewicht pro Flächeneinheit eines saugfähigen Materials hat, das den Saugkörper (40) konfiguriert; und
einen zweiten Bereich (42), der ein geringeres Gewicht pro Flächeneinheit des saugfähigen Materials als der erste Bereich (41) hat,
wobei sich der zweite Bereich (42) entlang der Längsrichtung des saugfähigen Artikels erstreckt,
**dadurch gekennzeichnet, dass** der zweite Bereich (42) zwischen ersten Bereichen (41) in der Breitenrichtung des saugfähigen Artikels sandwichartig angeordnet ist und die Verbundeinheit (70) in dem zweiten Bereich (42) ausgebildet ist.

2. Saugfähiger Artikel gemäß Anspruch 1, wobei sich der zweite Bereich (42) entlang der Breitenrichtung des saugfähigen Artikels erstreckt.

3. Saugfähiger Artikel gemäß Anspruch 1, wobei eine Breite (W1) senkrecht zur Erstreckungsrichtung des zweiten Bereichs (42) mindestens gleich einer Breite (12) senkrecht zur Erstreckungsrichtung des Verbundbereichs (70) ist.

4. Saugfähiger Artikel gemäß Anspruch 1, ferner umfassend eine Leckage-Verhinderungseinheit (80), die entlang der Längsrichtung des saugfähigen Artikels angeordnet ist, mit einem seilartigen Körper (81), der bei einer Dehnung an den Stirnseiten des Saugkörpers (40) in der Längsrichtung des saugfähigen Artikels über eine Elastizität verfügt, wobei sich der zweite Bereich (42) mit der Leckage-Verhinderungseinheit (80) relativ zur Dickenrichtung des saugfähigen Artikels nicht überlappt.

5. Saugfähige Artikel gemäß Anspruch 1, wobei eine Klebeeinheit (60), die über eine Klebefähigkeit verfügt, in der Rückschicht (20) auf der der Deckschicht (10) entgegengesetzten Oberfläche vorgesehen ist und sich der zweite Bereich (42) relativ zur Dickenrichtung des saugfähigen Artikels mit der Klebeeinheit (60) überlappt.

6. Saugfähiger Artikel gemäß Anspruch 1, wobei der erste Bereich (41) umfasst:
einen inneren Bereich (41A), der relativ zur Breitenrichtung des saugfähigen Artikels weiter innen angeordnet ist als der zweite Bereich (42); und
einen inneren Bereich (41B), der relativ zur Breitenrichtung des saugfähigen Artikels weiter außen angeordnet ist als der zweite Bereich (42) und der ein geringeres Gewicht pro Flächeneinheit des saugfähigen Materials als der innere Bereich (41A) hat.

7. Verfahren zum Herstellen eines saugfähigen Artikels, der eine flüssigkeitsdurchlässige Deckschicht (10), eine flüssigkeitsundurchlässige Rückschicht (20) und einen Saugkörper (40), der zwischen der Deckschicht (10) und der Rückschicht (20) vorgesehen ist, umfasst, wobei das Verfahren umfasst:
einen Schritt A zum Ausbilden des Saugkörpers (40), wobei ein erster Bereich (41) ein vorbestimmtes Gewicht pro Flächeneinheit eines saugfähigen Materials hat, das den Saugkörper (40) konfiguriert, und ein zweiter Bereich (42) ein geringeres Gewicht pro Flächeneinheit des saugfähigen Materials als der erste Bereich (41) hat; und
einen Schritt B zum Ausbilden einer Verbundeinheit (70) durch Verbinden mindestens der Deckschicht (10) und des Saugkörpers (40), wobei
im Schritt A der zweite Bereich (42) entlang der Längsrichtung des saugfähigen Artikels ausgebildet ist, und
das Verfahren **dadurch gekennzeichnet ist, dass**:
der zweite Bereich (42) so ausgebildet ist, dass er zwischen ersten Bereichen (41) in der Breitenrichtung des saugfähigen Artikels sandwichartig angeordnet ist, und
im Schritt B die Verbundeinheit (70) in dem zweiten Bereich (42) ausgebildet wird.

## Revendications

1. Article absorbant comprenant une feuille supérieure perméable aux liquides (10), une feuille arrière imperméable aux liquides (20), et un élément absorbant (40) prévu entre la feuille supérieure (10) et la feuille arrière (20), de sorte qu'une unité de jonction (70) soit formée dans laquelle au moins la feuille supérieure (10) et l'élément absorbant (40) sont joints, dans lequel l'élément absorbant (40) comprend :
une première région (41) ayant un poids prédéterminé par surface unitaire d'un matériau absorbant configurant l'élément absorbant (40) ; et
une deuxième région (42) ayant un poids par surface unitaire du matériau absorbant inférieur à celui de la première région (41),
la deuxième région (42) s'étendant le long de la direction longitudinale de l'article absorbant,
**caractérisé en ce que** la deuxième région (42) est prise en sandwich entre les premières régions (41) dans la direction de largeur de l'article absorbant, et l'unité de jonction (70) est formée dans la deuxième région (42).

2. Article absorbant selon la revendication 1, dans lequel la deuxième région (42) s'étend le long de la direction de largeur de l'article absorbant.

3. Article absorbant selon la revendication 1, dans lequel une largeur (W1) orthogonale à la direction d'extension de la deuxième région (42) est au moins équivalente à une largeur (W₂) orthogonale à la direction d'extension de l'unité de jonction (70).

4. Article absorbant selon la revendication 1, comprenant en outre une unité antifuite (80) disposée le long de la direction longitudinale de l'article absorbant avec un corps similaire à un cordon (81) présentant une élasticité dans la direction longitudinale de l'article absorbant étiré au niveau des côtés d'extrémité de l'élément absorbant (40), dans lequel la deuxième région (42) ne recouvre pas l'unité antifuite (80) en relation avec la direction d'épaisseur de l'article absorbant.

5. Article absorbant selon la revendication 1, dans lequel une unité adhésive (60) présentant une adhésivité est prévue sur la surface du côté opposé à la feuille supérieure (10) dans la feuille arrière (20), et la deuxième région (42) recouvre l'unité adhésive (60) en relation avec la direction d'épaisseur de l'article absorbant.

6. Article absorbant selon la revendication 1, dans lequel la première région (41) comprend :
une région intérieure (41A) positionnée plus loin vers l'intérieur que la deuxième région (42) en relation avec la direction de largeur de l'article absorbant ; et
une région intérieure (41 B) positionnée plus loin vers l'extérieur que la deuxième région (42) en relation avec la direction de largeur de l'article absorbant, et ayant un poids par surface unitaire du matériau absorbant inférieur à celui de la région intérieure (41A).

7. Procédé de fabrication d'un article absorbant qui comprend une feuille supérieure imperméable aux liquides (10), une feuille arrière imperméable aux liquides (20), et un élément absorbant (40) prévu entre la feuille supérieure (10) et la feuille arrière (20), le procédé comprenant :
une étape A de formation, dans l'élément absorbant (40), d'une première région (41) ayant un poids prédéterminé par surface unitaire d'un matériau absorbant configurant l'élément absorbant (40) et d'une deuxième région (42) ayant un poids par surface unitaire du matériau absorbant inférieur à celui de la première région (41) ; et
une étape B de formation d'une unité de jonction (70) en joignant au moins la feuille supérieure (10) et l'élément absorbant (40), dans lequel
à l'étape A, la deuxième région (42) est formée le long de la direction longitudinale de l'article absorbant, et
le procédé étant **caractérisé en ce que** :
la deuxième région (42) est formée de manière à être prise en sandwich entre les premières régions (41) dans la direction de largeur de l'article absorbant, et
à l'étape B, l'unité de jonction (70) est formée dans la deuxième région (42).
